# EUROPEAN PATENT APPLICATION

(11) **EP 4 722 200 A1**
(43) Date of publication of application: **08.04.2026**
(21) Application number: 25768497.7
(22) Date of filing: 10.02.2025
(51) Int. Cl.: C07D 251/18, G03F 7/004

(54) **COMPOUND, METHOD FOR PREPARING SAME, AND CROSSLINKING AGENT, PHOTOSENSITIVE RESIN COMPOSITION, CURED FILM, AND ELECTRONIC DEVICE, COMPRISING SAME**

(30) Priority: 05.03.2024 KR 20240031401
(71) Applicant: LG Chem, Ltd., Seoul 07336 (KR)
(72) Inventor: LE, Duy Hieu, Daejeon 34122 (KR); HWANG, Seung Yeon, Daejeon 34122 (KR); JEONG, Jaemyeng, Daejeon 34122 (KR); LEE, Hoyong, Daejeon 34122 (KR)
(74) Representative: Goddar, Heinz J.
(86) International application number: PCT/KR2025/001893
(87) International publication number: WO 2025/187973

(57) **Abstract**

The present disclosure relates to a compound with a novel structure, a method for preparing the same, a crosslinking agent, a photosensitive resin composition, a cured layer, and an electronic device comprising the same.

## Description

### [TECHNICAL FIELD]

### Cross-Reference to Related Applications

This application claims the benefit of priority based on Korean Patent Application No. 10-2024-0031401, filed on March 5, 2024, and all contents disclosed in the document of the said Korean patent application are incorporated as part of this specification.

The present disclosure relates to a compound with excellent heat resistance and tensile properties, a method for preparing the same, a crosslinking agent, a photosensitive resin composition, a cured layer, and an electronic device comprising the same.

### [BACKGROUND ART]

Photosensitive resins are representative functional polymer materials that have been commercialized in the production of various precision electronic and information industry products and are currently being importantly used in advanced technology industries, particularly in the production of semiconductors and displays.

Generally, a photosensitive resin refers to a polymer compound that undergoes chemical changes in its molecular structure in a short time upon light irradiation, resulting in changes in properties such as solubility in specific solvents, coloration, and curing. Using photosensitive resins enables fine precision processing, significant savings in energy and raw materials compared to thermal reaction processes, and quick and accurate work in small installation spaces, making them widely used in various precision electronic and information industry fields such as advanced printing, semiconductor production, display production, and light-curing surface coating materials.

Meanwhile, as electronic devices have recently become more integrated and miniaturized, there is a demand for photosensitive resins that can minimize defect rates and improve processing efficiency and resolution. Accordingly, methods using polyimide or polybenzoxazole with strong resistance to external heat, chemicals, and impact as photosensitive resins have been introduced.

However, the more processability (especially developability in lithography processes) is imparted, the more the film quality of the cured product tends to break easily, indicating a need for improvement in tensile properties to ensure reliability against external physical stimuli.

For this purpose, the use of epoxy comprising alkylene oxide groups is well known. However, the higher the proportion of epoxy comprising alkylene oxide groups in the composition, the higher the overall content of flexible functional groups in the composition, resulting in a problem of lowering the glass transition temperature (Tg) of the final cured layer.

Therefore, there is a demand for the development of a composition that can enhance tensile properties without lowering the glass transition temperature.

### [DETAILED DESCRIPTION OF THE INVENTION]

### [Technical Problem]

The present disclosure aims to provide a compound with excellent heat resistance and tensile properties.

Additionally, the present disclosure aims to provide a method for preparing the said compound.

Furthermore, the present disclosure aims to provide a crosslinking agent comprising the said compound.

Moreover, the present disclosure aims to provide a photosensitive resin composition comprising the said compound.

Additionally, the present disclosure aims to provide a cured layer and an electronic device manufactured using the said compound or photosensitive resin composition.

### [Technical Solution]

In this specification, a compound represented by chemical formula 1 is provided.

This specification also provides a method for preparing a compound comprising the steps of reacting a compound represented by chemical formula 3 with dicyandiamide to produce a compound represented by chemical formula 4; reacting the compound represented by chemical formula 4 with formaldehyde to produce a compound represented by chemical formula 5; and reacting the compound represented by chemical formula 5 with alcohol.

Furthermore, a crosslinking agent comprising the said compound is provided in this specification.

Additionally, a photosensitive resin composition comprising the said compound is provided in this specification.

Moreover, a cured layer comprising the cured product of the said photosensitive resin composition is provided in this specification.

Furthermore, an electronic device comprising the said cured layer is provided in this specification.

Hereinafter, the compound, its method for preparing, the crosslinking agent, the photosensitive resin composition, the cured layer, and the electronic device according to specific embodiments of the invention comprising the same will be described in more detail.

In this specification, when a part is described as "comprising" a certain component, it means that other components can be further included unless specifically stated otherwise.

In this specification, examples of substituents are described below, but are not limited thereto.

In this specification, the term "substitution" means that a functional group other than hydrogen atoms is bonded in the compound, and the position of substitution is not limited as long as it is a position where hydrogen atoms can be substituted, and when two or more substitutions occur, two or more substituents can be the same or different from each other.

In this specification, the term "substituted or unsubstituted" means substituted or unsubstituted with one or more substituents selected from the group consisting of hydrogen; halogen group; cyano group; nitro group; hydroxy group; carbonyl group; ester group; imide group; amide group; amino group; carboxyl group; sulfonic acid group; sulfonamide group; phosphine oxide group; alkoxy group; aryloxy group; alkylthioxy group; arylthioxy group; alkylsulfoxy group; arylsulfoxy group; silyl group; alkyl group; cycloalkyl group; alkenyl group; aryl group; aralkyl group; aralkenyl group; alkylaryl group; arylphosphine group; or heterocyclic group comprising one or more of N, O, and S atoms, or being unsubstituted or substituted with a substituent group to which two or more substituent groups of the above-exemplified substituent groups are linked. For example, "a substituent with two or more substituents connected" can be a biphenyl group. That is, the biphenyl group can be interpreted as an aryl group or a substituent with two phenyl groups connected.

In this specification, 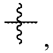 or 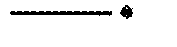 means a bond connecting to other substituents, and direct bond means a case where there is no separate atom in the part represented by L.

In this specification, examples of halogen groups include fluorine, chlorine, bromine, or iodine.

In this specification, alkyl is a monovalent functional group derived from alkane, which can be linear or branched, and the number of carbon atoms in the linear alkyl is not particularly limited but is preferably 1 to 20. Additionally, the number of carbon atoms in the branched alkyl is 3 to 20. Specific examples of alkyl include methyl, ethyl, propyl, n-propyl, isopropyl, butyl, n-butyl, isobutyl, tert-butyl, sec-butyl, 1-methyl-butyl, 1-ethyl-butyl, pentyl, n-pentyl, isopentyl, neopentyl, tert-pentyl, hexyl, n-hexyl, 1-methylpentyl, 2-methylpentyl, 4-methyl-2-pentyl, 3,3-dimethylbutyl, 2-ethylbutyl, heptyl, n-heptyl, 1-methylhexyl, octyl, n-octyl, tert-octyl, 1-methylheptyl, 2-ethylhexyl, 2-propylpentyl, n-nonyl, 2,2-dimethylheptyl, 1-ethyl-propyl, 1,1-dimethyl-propyl, isohexyl, 4-methylhexyl, 5-methylhexyl, 2,6-dimethylheptan-4-yl, but is not limited to these. The alkyl can be substituted or unsubstituted, and in the case of substitution, examples of substituents are as described above.

In this specification, cycloalkyl is a monovalent functional group derived from cycloalkane, which can be monocyclic or polycyclic and is not particularly limited, but the number of carbon atoms is 3 to 20. According to another embodiment, the number of carbon atoms in the cycloalkyl is 3 to 10. Specifically, examples thereof include cyclopropyl, cyclobutyl, cyclopentyl, 3-methylcyclopentyl, 2,3-dimethylcyclopentyl, cyclohexyl, 3-methylcyclohexyl, 4-methylcyclohexyl, 2,3-dimethylcyclohexyl, 3,4,5-trimethylcyclohexyl, 4-tert-butylcyclohexyl, cycloheptyl, cyclooctyl, bicyclo[2,2,1]heptyl, but is not limited to these. The cycloalkyl can be substituted or unsubstituted, and in the case of substitution, examples of substituents are as described above.

In this specification, alkoxy is a functional group in which the alkyl described above is bonded to one terminal of an ether group (-O-), and the description of the alkyl described above can be applied except that they are functional groups bonded with an ether group (-O-). For example, it can be linear, branched, or cyclic. The number of carbon atoms in the alkoxy is not particularly limited, but it is preferably 1 to 20. Specifically, examples thereof include methoxy, ethoxy, n-propoxy, isopropoxy, i-propoxy, n-butoxy, isobutoxy, tert-butoxy, sec-butoxy, n-pentyloxy, neopentyloxy, isopentoxy, n-hexyloxy, 3,3-dimethylbutyl oxy, 2-ethylbutyl oxy, n-octyl oxy, n-nonyl oxy, n-decyl oxy, cycloheptyloxy, benzyl oxy, p-methylbenzyl oxy, but is not limited to these. The alkoxy can be substituted or unsubstituted.

In this specification, aryl is a monovalent functional group derived from arene, which is not particularly limited, but is preferably 6 to 20 carbon atoms and can be a monocyclic aryl group or a polycyclic aryl group. The monocyclic aryl can be phenyl, biphenyl, terphenyl, but is not limited to these. The polycyclic aryl can be naphthyl, anthracenyl, phenanthryl, pyrenyl, perylenyl, chrysene, fluorenyl, but is not limited to these. The aryl can be substituted or unsubstituted, and in the case of substitution, examples of substituents are as described above.

In this specification, alkylene is a divalent functional group derived from alkane, and the description of the alkyl described above can be applied except that they are divalent functional groups. For example, it can be linear or branched, such as methylene, ethylene, propylene, isobutylene, sec-butylene, tert-butylene, pentylene, hexylene. The alkylene can be substituted or unsubstituted, and in the case of substitution, examples of substituents are as described above.

In this specification, arylene is a divalent functional group derived from arene, and the description of the aryl described above can be applied except that they are divalent functional groups. For example, phenylene, biphenylene, terphenylene, naphthylene, fluorenyl, pyrenyl, phenanthrylene, perylene, tetracenyl, anthracenyl can be included. The arylene can be substituted or unsubstituted, and in the case of substitution, examples of substituents are as described above.

In this specification, cycloalkylene is a divalent functional group derived from cycloalkane, and the description of the cycloalkyl described above can be applied except that they are divalent functional groups. The cycloalkylene can be substituted or unsubstituted, and in the case of substitution, examples of substituents are as described above.

In this specification, "direct bond" means that there is no atom or atomic group at the relevant position, and it is connected by a bond line.

According to one embodiment of the invention, a compound represented by chemical formula 1 below can be provided. 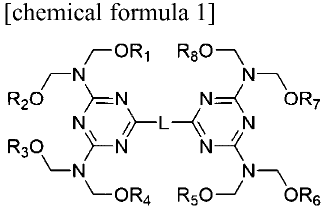 In the chemical formula 1 above,
R₁ to R₈ are the same or different from each other, each independently being one of alkyl, arylalkyl, or alkoxyarylalkyl, and
L is a functional group represented by chemical formula 2 below, 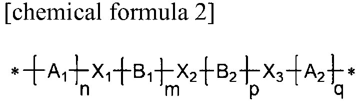 in the chemical formula 2 above,
   X₁, X₂, X₃ are the same or different from each other, each independently being one of direct bond, -O-, -COO-, -CONR₉-, -O(CO)O-, or -NR₁₀(CO)NR₁₁-, and
   R₉ to R₁₁ are the same or different from each other, each independently being one of hydrogen, alkyl, cycloalkyl, or aryl, and
   A₁, A₂, B₁, and B₂ are the same or different from each other, each independently being one of alkylene, cycloalkylene, or arylene, and
   n, m, p, and q are the same or different from each other, each independently being an integer of 0 or 1, and at least one of n, m, p, and q is 1.

The inventors of the present disclosure confirmed through experiments that the compound represented by chemical formula 1 can be used as a crosslinking agent because it can react with electron-rich systems in heat or acid due to its chemical structure, and since the compound represented by chemical formula 1 has many crosslinking sites (8 sites), the tensile properties of the cured layer comprising the compound represented by chemical formula 1 can be significantly improved, and the reduction of the glass transition temperature can be suppressed, and completed the invention.

In the chemical formula 1 above, R1 to R8 are the same or different from each other, each independently being one of alkyl, arylalkyl, or alkoxyarylalkyl. The arylalkyl means a functional group in which hydrogen atoms contained in alkyl are substituted with aryl. Also, the alkoxyarylalkyl means a functional group in which hydrogen atoms contained in alkyl are substituted with alkoxyaryl, and the alkoxyaryl means a functional group in which hydrogen atoms contained in aryl are substituted with alkoxy.

Specifically, in the chemical formula 1 above, R₁ to R₈ are the same or different from each other, each independently being one of alkyl with 1 to 5 carbon atoms, arylalkyl with 7 to 20 carbon atoms, or alkoxyarylalkyl with 8 to 30 carbon atoms.

Meanwhile, in the chemical formula 2 above, X₁, X₂, X₃ are the same or different from each other, each independently being one of direct bond, -O-, -COO-, -CONR₉-, - O(CO)O-, or -NR₁₀(CO)NR₁₁-. The R₉ to R₁₁ described in the -CONR₉-, or - R₁₀(CO)NR₁₁- are the same or different from each other, each independently being one of hydrogen, alkyl, cycloalkyl, or aryl. More specifically, the R₉ can be hydrogen, methyl, or ethyl. Additionally, the R₁₀ and R₁₁ can be the same or different from each other, each independently being one of hydrogen or methyl.

In the chemical formula 2 above, X₁, X₂, X₃ can all be the same and be a direct bond.

Also, in the chemical formula 2 above, at least one of X₁, X₂, X₃ can be one of - O-, -COO-, -CONR₉-, -O(CO)O-, or -NR₁₀(CO)NR₁₁-. That is, one or more of X₁, X₂, X₃ can be one of -O-, -COO-, -CONR₉-, -O(CO)O-, or -NR₁₀(CO)NR₁₁-. This can ensure molecular flexibility in terms of molecular structure, increase packing between polymer chains, significantly improve the tensile properties of the cured layer comprising the compound represented by chemical formula 1, and suppress the reduction of the glass transition temperature.

In the chemical formula 2 above, specific examples of X₁, X₂, X₃ are not particularly limited, but for example, they can be one of -O-, -COO-, -CONH-, - CON(CH₃)-, -CON(CH₂CH₃)-, -O(CO)O-, -NH(CO)NH-, or -N(CH₃)(CO)N(CH₃)-.

On the other hand, if at least one of X₁, X₂, X₃ in the chemical formula 2 is - NR₁₂- (where R₁₂ is one of hydrogen, alkyl, cycloalkyl, alkoxyalkyl, or aryl), the manufacturing process becomes complicated, and as the number of amine groups increases, reliability (especially heat/moisture resistance) can be reduced.

Meanwhile, in the chemical formula 2 above, A₁, A₂, B₁, and B₂ are the same or different from each other, each independently being one of alkylene, cycloalkylene, or arylene.

Specific examples of A₁, A₂, B₁, and B₂ are not particularly limited, but for example, they can be one of methylene, ethylene, normal propylene, 1,3-butanediyl, cyclohexylene, 1,3-phenylene, 5-methyl-1,3-phenylene, 2,5-dimethyl-1,4-phenylene, 1,5-naphthylene, or 1,6-naphthylene. This can ensure molecular flexibility in terms of molecular structure, strengthen pi-pi interaction, significantly improve the tensile properties of the cured layer comprising the compound represented by chemical formula 1, and suppress the reduction of the glass transition temperature.

Meanwhile, in the chemical formula 2 above, n, m, p, and q are the same or different from each other, each independently being an integer of 0 or 1, and at least one of n, m, p, and q can be 1. That is, one or more of n, m, p, and q can be 1.

In the chemical formula 2 above, (n+m+p+q) can be an integer from 2 to 4, or 3 to 4, or 4. This can ensure molecular flexibility in terms of molecular structure, strengthen pi-pi interaction, significantly improve the tensile properties of the cured layer comprising the compound represented by chemical formula 1, and suppress the reduction of the glass transition temperature.

More specifically, the functional group represented by chemical formula 2 can be any one selected from the group consisting of the following. However, specific examples of the functional group represented by chemical formula 2 are not limited to the group consisting of the following.

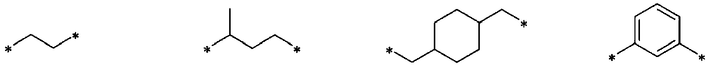

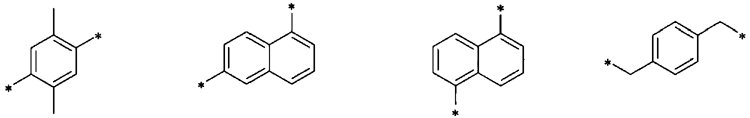

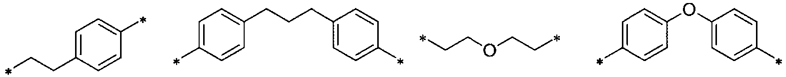

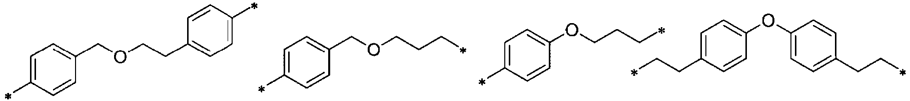

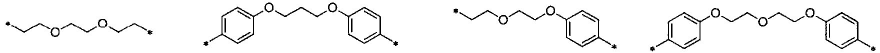

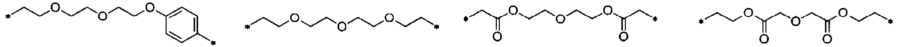

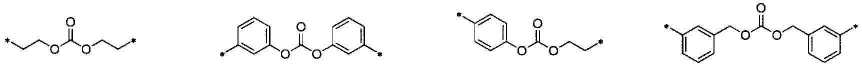

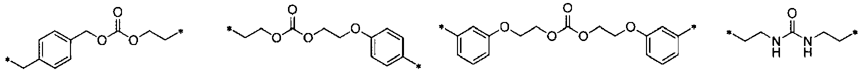

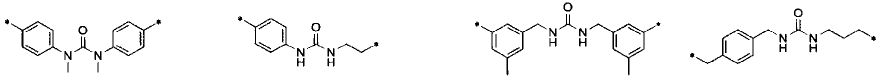

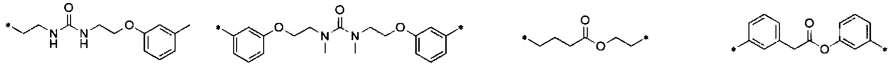

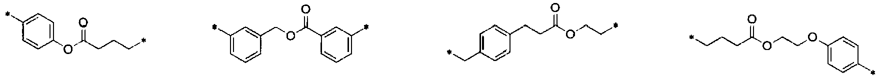

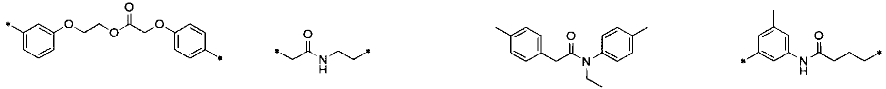

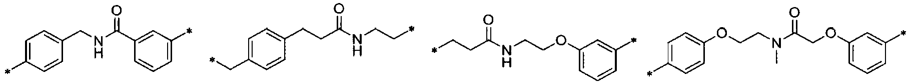

Meanwhile, the compound represented by chemical formula 1 can comprise a compound represented by any one of chemical formula 1-1 to chemical formula 1-12 below. However, specific examples of the compound represented by chemical formula 1 are not limited to the compound represented by any one of chemical formula 1-1 to chemical formula 1-12 below.

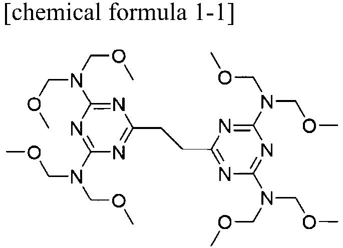

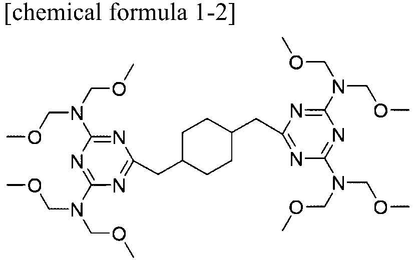

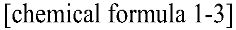

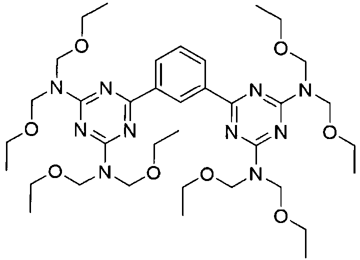

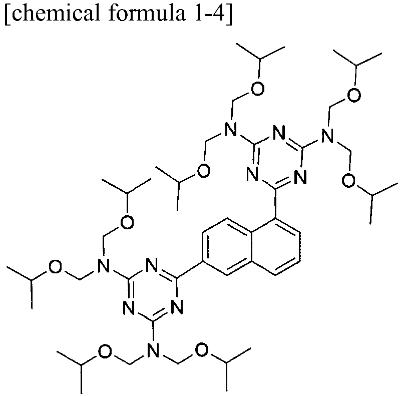

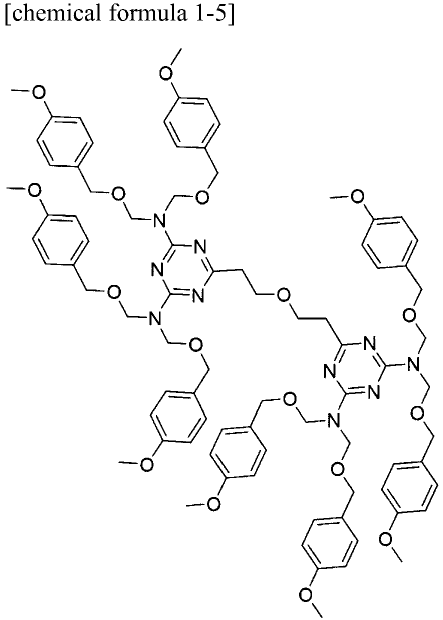

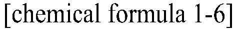

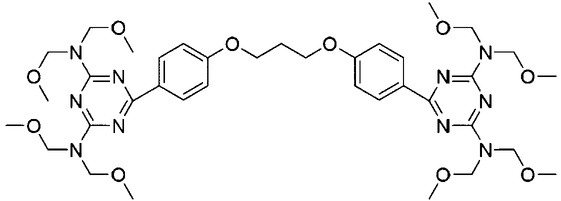

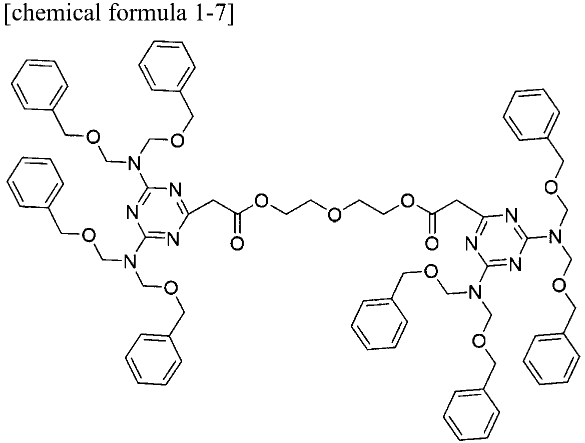

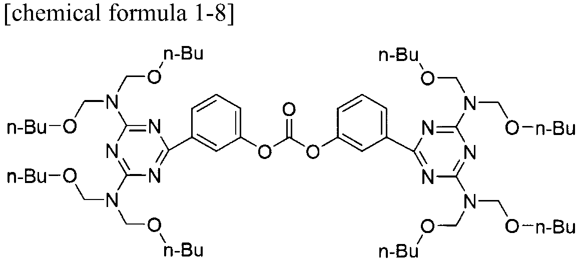

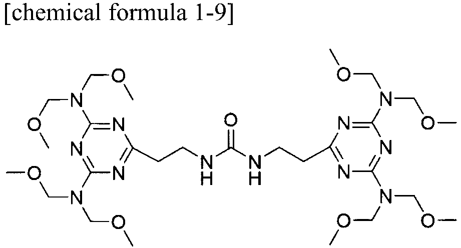

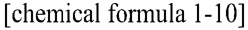

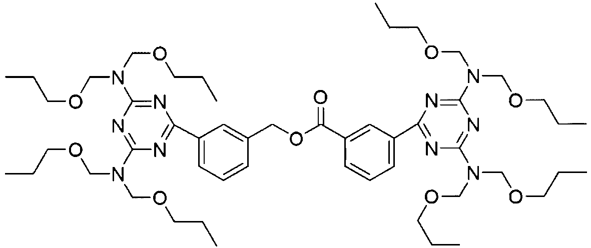

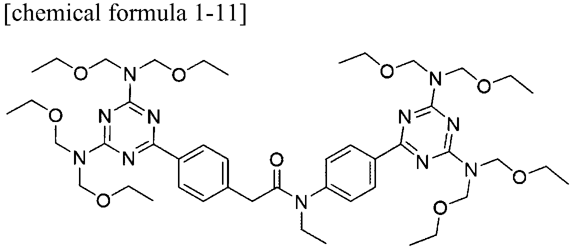

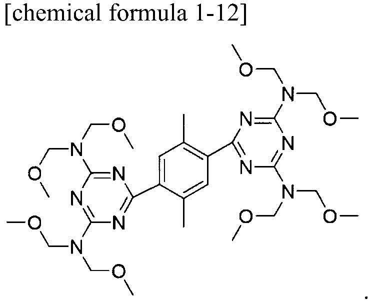

The compound of the embodiment has a Formaldehyde index of 38 ppm or less, 37 ppm or less, 32 ppm or less, 29 ppm or less, 26 ppm or less, 25 ppm or less, 24 ppm or less, 23 ppm or less, 21 ppm or less, 20 ppm or less, 19 ppm or less, 18 ppm or less, 17 ppm or less, 1 ppm or more, 1 ppm to 38 ppm, 1 ppm to 37 ppm, 1 ppm to 32 ppm, 1 ppm to 29 ppm, 1 ppm to 26 ppm, 1 ppm to 25 ppm, 1 ppm to 24 ppm, 1 ppm to 23 ppm, 1 ppm to 21 ppm, 1 ppm to 20 ppm, 1 ppm to 19 ppm, 1 ppm to 18 ppm, or 1 ppm to 17 ppm according to EPA Method 8315A.

The method for measuring the Formaldehyde index according to EPA Method 8315A is not particularly limited, and for example, it can be measured using the EPA (US Environmental Protection Agency) Method 8315A (SW-846) method.

Formaldehyde is a substance with a very irritating smell that irritates the nose and eyes and has harmful components to humans and the natural environment. Recently, it has been suspected as a carcinogen, attracting worldwide attention, and legal regulations are being strengthened, requiring that formaldehyde should not be included in the manufacturing process of the compound. As the Formaldehyde index according to EPA Method 8315A is reduced to 38 ppm or less, the compound of the embodiment enables the synthesis of an environmentally friendly compound.

Meanwhile, according to another embodiment of the invention, a method for preparing a compound comprising the steps of reacting a compound represented by chemical formula 3 with dicyandiamide to produce a compound represented by chemical formula 4; reacting the compound represented by chemical formula 4 with formaldehyde to produce a compound represented by chemical formula 5; and reacting the compound represented by chemical formula 5 with alcohol is provided.

[chemical formula 3] NC-L-CN

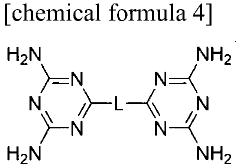

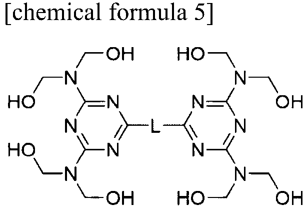

in the chemical formula 3 to 5 above,
L is a functional group represented by chemical formula 2 below, 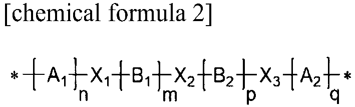 in the chemical formula 2 above,
X₁, X₂, X₃ are the same or different from each other, each independently being one of direct bond, -O-, -COO-, -CONR₉-, -O(CO)O-, or -NR₁₀(CO)NR₁₁-, and
R₉ to R₁₁ are the same or different from each other, each independently being one of hydrogen, alkyl, cycloalkyl, or aryl, and
A₁, A₂, B₁, and B₂ are the same or different from each other, each independently being one of alkylene, cycloalkylene, or arylene, and
n, m, p, and q are the same or different from each other, each independently being an integer of 0 or 1, and at least one of n, m, p, and q is 1.

The content related to the chemical formula 2 is incorporated in the content described above regarding the embodiment.

In the step of reacting the compound represented by chemical formula 3 with dicyandiamide to produce the compound represented by chemical formula 4, dicyandiamide can be added in sufficient excess compared to the compound represented by chemical formula 3 to ensure that dicyandiamide reacts with all terminal cyano groups of the compound represented by chemical formula 3.

Additionally, in the step of reacting the compound represented by chemical formula 3 with dicyandiamide to produce the compound represented by chemical formula 4, the reaction between the compound represented by chemical formula 3 and dicyandiamide can proceed under basic conditions. The example of the base is not particularly limited, but for example, KOH can be used. Also, the reaction between the compound represented by chemical formula 3 and dicyandiamide can proceed under solvent conditions. The example of the solvent is not particularly limited, but for example, 2-Methoxyethanol can be used.

Meanwhile, in the step of reacting the compound represented by chemical formula 4 with formaldehyde to produce the compound represented by chemical formula 5, formaldehyde can be added in sufficient excess compared to the compound represented by chemical formula 4 to ensure that formaldehyde reacts with all terminal amino groups of the compound represented by chemical formula 4.

Additionally, in the step of reacting the compound represented by chemical formula 4 with formaldehyde to produce the compound represented by chemical formula 5, the reaction between the compound represented by chemical formula 4 and formaldehyde can proceed under basic conditions. The example of the base is not particularly limited, but for example, NaHCO₃ can be used.

Meanwhile, in the step of reacting the compound represented by chemical formula 5 with alcohol, the alcohol can comprise a compound represented by chemical formula 6 below.

[chemical formula 6] R₁₃-OH

in the chemical formula 6 above,
R₁₃ is one of alkyl, arylalkyl, or alkoxyarylalkyl.

In the chemical formula 6 above, R₁₃ can be one of alkyl, arylalkyl, or alkoxyarylalkyl. The arylalkyl means a functional group in which hydrogen atoms contained in alkyl are substituted with aryl. Also, the alkoxyarylalkyl means a functional group in which hydrogen atoms contained in alkyl are substituted with alkoxyaryl, and the alkoxyaryl means a functional group in which hydrogen atoms contained in aryl are substituted with alkoxy.

Specifically, in the chemical formula 6 above, R₁₃ can be one of alkyl with 1 to 5 carbon atoms, arylalkyl with 7 to 20 carbon atoms, or alkoxyarylalkyl with 8 to 30 carbon atoms.

Additionally, in the step of reacting the compound represented by chemical formula 5 with alcohol, alcohol can be added in sufficient excess compared to the compound represented by chemical formula 5 to ensure that alcohol reacts with all terminal hydroxy groups of the compound represented by chemical formula 5.

Additionally, in the step of reacting the compound represented by chemical formula 5 with alcohol, the reaction between the compound represented by chemical formula 5 and alcohol can proceed under acidic conditions. The example of the acid is not particularly limited, but for example, HCl can be used.

Additionally, the reaction between the compound represented by chemical formula 5 and alcohol can proceed under solvent conditions. The example of the solvent is not particularly limited, but for example, Toluene can be used.

Meanwhile, after the step of reacting the compound represented by chemical formula 5 with alcohol, a neutralization step can be further proceeded. Since the step of reacting the compound represented by chemical formula 5 with alcohol proceeds under acidic conditions, a base can be added in the neutralization step, and the example of the base is not particularly limited, but for example, NaOH can be used.

After synthesizing the compound represented by chemical formula 1 of the embodiment through the method for preparing the compound of the other embodiment, if necessary, additional steps such as filtration or drying can be carried out. The content related to the filtration step and drying step can be applied without limitation to various methods, equipment, and conditions that have been widely used in the conventional compound synthesis field.

Meanwhile, according to another embodiment of the invention, a crosslinking agent comprising the compound of the embodiment can be provided.

The content related to the compound is incorporated in the content described above regarding the embodiment. Various functional groups contained in the compound can form a crosslinking structure with functional groups comprised in the polyimide during the curing process mediated by heat or acid, and due to this crosslinking structure, it can form a crosslinking structure with other adjacent polyimides. Accordingly, the photosensitive resin composition comprising the crosslinking agent of the other embodiment can significantly increase tensile strength while maintaining high resistance to various chemicals and heat.

Meanwhile, according to another embodiment of the invention, a photosensitive resin composition comprising the compound of the embodiment can be provided.

The content related to the compound is incorporated in the content described above regarding the embodiment.

The photosensitive resin composition can further comprise a binder resin. The binder resin is not particularly limited as long as it can exhibit the properties such as strength and developability of the film manufactured from the resin composition. However, as an example, polyimide or polybenzoxazole can be used.

Additionally, the photosensitive resin composition can further comprise a photoinitiator. The photoinitiator is not particularly limited as long as it is an initiator that generates radicals upon exposure to light to trigger crosslinking, but for example, it can be one or more selected from the group consisting of benzophenone-based compounds, acetophenone-based compounds, bisimidazole-based compounds, triazine-based compounds, and oxime-based compounds.

The photosensitive resin composition can further comprise a solvent. The solvent can be applied without special limitation as a compound known to enable the formation of a photosensitive resin composition layer in the technical field to which the present disclosure belongs. Non-limiting examples of the solvent can be one or more compounds selected from the group consisting of esters, ethers, ketones, aromatic hydrocarbons, and sulfoxides.

Additionally, the photosensitive resin composition can further comprise one or two or more additives selected from the group consisting of antioxidants, photoinitiator enhancers, curing accelerators, adhesion promoters, surfactants, thermal polymerization inhibitors, ultraviolet absorbers, dispersants, and leveling agents.

Meanwhile, according to another embodiment of the invention, a cured layer comprising the cured product of the photosensitive resin composition can be provided. The cured product refers to the material obtained through the curing process of the photosensitive resin composition of the other embodiment. The content related to the photosensitive resin composition is incorporated in the content described above regarding the other embodiment. The cured layer can comprise both an organic insulating film and a photosensitive pattern.

The cured layer exhibits excellent heat resistance and tensile properties and can be applied to the insulating film of semiconductor devices, interlayer insulating films for rewiring layers, and the like. Furthermore, the cured layer can be applied to photoresists, etching resists, solder top resists, and the like.

Meanwhile, according to another embodiment of the invention, an electronic device comprising the cured layer of the embodiment can be provided. The content related to the cured layer is incorporated in the content described above regarding the other embodiment.

The electronic device comprising an organic insulating film or photosensitive pattern formed from the photosensitive resin composition containing the compound of the embodiment can implement excellent performance such as high resolution and high sensitivity and exhibit excellent film properties and high mechanical properties, as well as excellent heat resistance, for example, even when used for a long time or exposed to high-temperature conditions for a long time, the adhesion of the organic insulating film or photosensitive pattern can be maintained firmly without deterioration.

Examples of the electronic device include semiconductor devices.

### [Advantageous Effects]

According to the present disclosure, a compound with excellent heat resistance and tensile properties, and a crosslinking agent, photosensitive resin composition, cured layer, and electronic device comprising the same can be provided.

### [DETAILED DESCRIPTION OF THE EMBODIMENTS]

The invention will be described in more detail in the following examples. However, the following examples are merely illustrative of the present disclosure, and the content of the present disclosure is not limited by the following examples.

### <MANUFACTURING Example 1>: Synthesis of compounds 1 to 12

### (1) Preparation of compound 1

Compound 1 was synthesized through the reaction shown in [Reaction Formula 1] below.

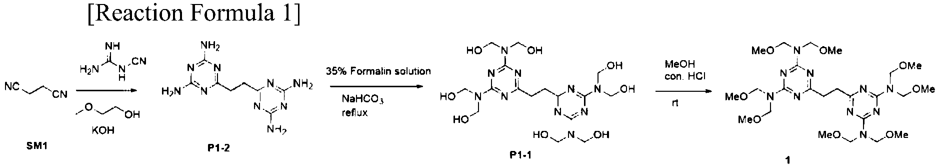

In a 500ml round-bottom flask, 8g (0.1mol) of Succinonitrile (SM1, CAS:110-61-2), 17.6g (1mol, 2.1eq) of Dicyandiamide (CAS:461-58-5), 0.56g (0.01mol, 0.1eq) of KOH, and 80ml of 2-Methoxyethanol were added and refluxed for 12 hours. After the reaction was completed, it was further stirred at room temperature for 1 hour, and P1-2 compound was obtained through filtration.

In a 500ml round-bottom flask, the obtained P1-2 compound, 0.84g (0.01mol, 0.1eq) of NaHCO₃, and 80ml (1mol, 10eq) of 35% formaldehyde aqueous solution were added and stirred at 80 °C for 12 hours. After the reaction was completed, 40ml of acetonitrile was slowly added. After the reaction was completed, it was further stirred at room temperature for 1 hour, and P1-1 compound was obtained through filtration.

In a 250ml round-bottom flask, the obtained P1-1 compound and 80ml of Methanol were added and cooled to 5 °C, and then 0.12ml (0.01mol, 0.1eq) of 37% Hydrochloric acid was added. After raising the temperature to room temperature, it was stirred until completely dissolved. After the reaction was completed, 40ml of 5% NaOH solution was slowly added. After stirring for 1 hour, a solid was formed. After the filtration process, it was dried using an 80 °C vacuum oven, and 12g of compound 1 was obtained. (Yield 12%)
¹H NMR (500MHz, Acetone-D6, ppm): 5.2(s, 16H), 3.3-3.5(broad, 24H), 2.5(s, 4H)
HRMS [M+H]⁺: 601.3381

### (2) Preparation of compound 2

Compound 2 was synthesized through the reaction shown in [Reaction Formula 2] below.

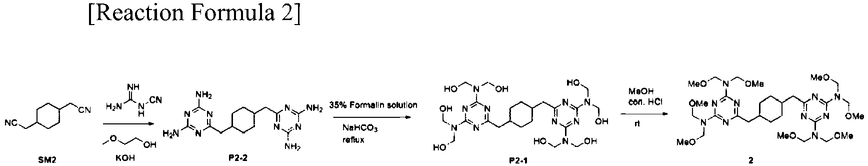

Specifically, as shown in [Reaction Formula 2], except for using 16g (0.1mol) of SM2 compound (CAS: 90872-59-6) instead of 8g (0.1mol) of Succinonitrile (SM1, CAS:110-61-2), 15g of compound2 was obtained by the same method as the preparation example of compound 1. (Yield 21%)
¹H NMR (500MHz, Acetone-D6, ppm): 5.1(s, 16H), 3.2-3.4(broad, 24H), 2.52(d, 4H), 1.79(multiplet, 2H), 1.6 (m, 4H), 1.4 (m, 4H)
HRMS [M+H]⁺: 683.4162

### (3) Preparation of compound 3

Compound 3 was synthesized through the reaction shown in [Reaction Formula 3] below.

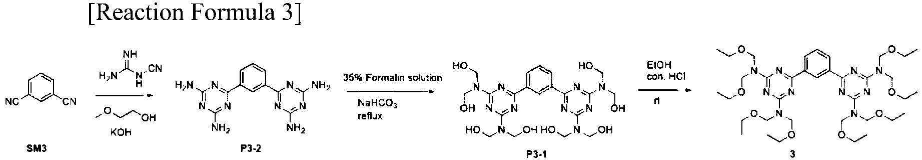

Specifically, as shown in [Reaction Formula 3], except for using 13g (0.1mol) of SM3 compound (CAS: 626-17-5) instead of 8g (0.1mol) of Succinonitrile (SM1, CAS:110-61-2), 15g of P3-1 compound was synthesized by the same method as the preparation example of compound 1.

In a 250ml round-bottom flask, the obtained P3-1 compound and 100ml of Ethanol were added and cooled to 5 °C, and then 0.24ml (0.02mol, 0.2eq) of 37% Hydrochloric acid was added. After raising the temperature to room temperature, it was stirred until completely dissolved. After the reaction was completed, 50ml of 5% NaOH solution was slowly added. After stirring for 1 hour, a solid was formed. After the filtration process, it was dried using an 80 °C vacuum oven, and 18.2g of compound 3 was obtained. (Yield 24%)
¹H NMR (500MHz, Acetone-D6, ppm): 8.45 (d, 2H), 7.87 (s, 1H), 7.65 (t, 1H), 5.4(s, 16H), 3.5(q, 16H), 1.1(t, 24H)
HRMS [M+H]⁺: 761.4626

### (4) Preparation of compound 4

Compound 4 was synthesized through the reaction shown in [Reaction Formula 4] below.

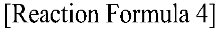

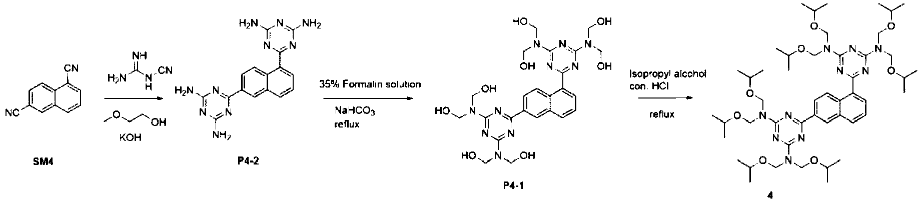

Specifically, as shown in [Reaction Formula 4], except for using 18g (0.1mol) of SM4 compound (CAS: 46289-40-1) instead of 8g (0.1mol) of Succinonitrile (SM1, CAS:110-61-2), P4-1 compound was synthesized by the same method as the preparation example of compound 1.

In a 500ml round-bottom flask, the obtained P4-1 compound and 100ml of isopropanol were added and cooled to 5 °C, and then 1.2ml (0.1 mol, 1eq) of 37% Hydrochloric acid was added. After raising the temperature to room temperature, it was stirred until completely dissolved. After the reaction was completed, 50ml of 5% NaOH solution was slowly added. After stirring for 1 hour, a solid was formed. After the filtration process, it was dried using an 80 °C vacuum oven, and 23g of compound 4 was obtained. (Yield 25%)

In a 500ml round-bottom flask, P4-1 compound was added with 100ml of isopropanol, cooled to 5°C, and 1.2ml (0.1 mol, 1eq) of 37% Hydrochloric acid was added. After raising the temperature to room temperature, it was stirred until completely dissolved. After the reaction was completed, 50ml of 5% NaOH solution was slowly added. After stirring for 1 hour, a solid was formed. After the filtration process, it was dried using an 80°C vacuum oven, and 23g (3 steps, total yield 25%) of compound 4 was obtained.
¹H NMR (500MHz, Acetone-D6, ppm): 8.3-8.4 (m, 2H), 8.1-8.2 (m, 4H), 5.5(s, 16H), 3.65(m, 8H), 1.2(d, 48H)
HRMS [M+H]⁺: 923.6035

### (5) Preparation of compound 5

Compound 5 was synthesized through the reaction shown in [Reaction Formula 5] below.

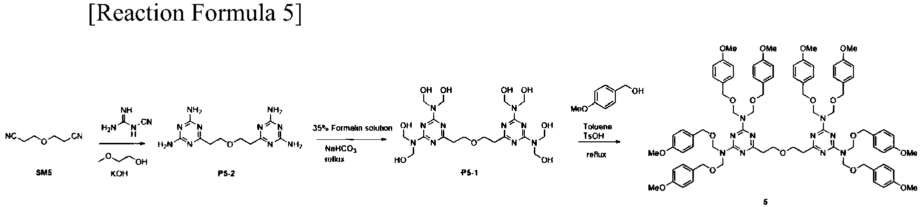

Specifically, as shown in [Reaction Formula 5], except for using 12.5g (0.1mol) of SM5 compound (CAS: 1656-48-0) instead of 8g (0.1mol) of Succinonitrile (SM1, CAS:110-61-2), P5-1 compound was synthesized by the same method as the preparation example of compound 1.

In a 500ml round-bottom flask, the obtained P5-1 compound, 138g (1mol, 10eq) of 4-Methoxy benzylalcohol, 1.92g (0.01mol, 0.1eq) of p-Toluenesulfonic acid, and 280ml of Toluene were added and refluxed. After the reaction was completed, 140ml of 5% NaOH solution was slowly added and neutralized. After extracting the Toluene layer, the solvent was removed to obtain an orange solid. After recrystallization with Ethanol, 22g of compound 5 was obtained. (Yield 15%)
¹H NMR (500MHz, Acetone-D6, ppm): 7.01 (d, 16H), 6.88 (d, 16H), 5.6 (s, 16H), 4.9 (s, 16H), 3.9 (s, 24H), 3.4 (t, 4H), 2.5 (t, 4H)
HRMS [M+H]⁺: 1493.6982

### (6) Preparation of compound 6

Compound 6 was synthesized through the reaction shown in [Reaction Formula 6] below.

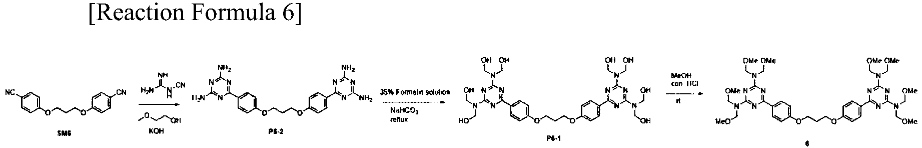

Specifically, as shown in [Reaction Formula 6], except for using 28g (0.1mol) of SM6 compound (CAS: 7476-06-4) instead of 8g (0.1mol) of Succinonitrile (SM1, CAS:110-61-2), 24g of compound 6 was obtained by the same method as the preparation example of compound 1. (Yield 30%)
¹H NMR (500MHz, Acetone-D6, ppm): 8.1 (d, 4H), 7.1 (d, 4H), 5.5 (s, 16H), 4.2 (t, 4H), 3.4 (s, 24H), 2.2 (m, 2H)
HRMS [M+H]⁺: 799.4062

### (7) Preparation of compound 7

Compound 7 was synthesized through the reaction shown in [Reaction Formula 7] below.

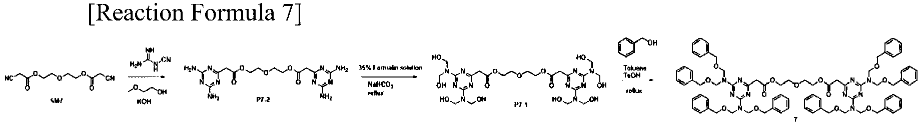

Specifically, as shown in [Reaction Formula 7], except for using 24g (0.1mol) of SM7 compound (CAS: 90870-40-9) instead of 8g (0.1mol) of Succinonitrile (SM1, CAS:110-61-2), P7-1 compound was synthesized by the same method as the preparation example of compound 1.

In a 500ml round-bottom flask, the obtained P7-1 compound, 108g (1mol, 10eq) of Benzylalcohol, 1.92g (0.01mol, 0.1eq) of p-Toluenesulfonic acid, and 220ml of Toluene were added and refluxed. After the reaction was completed, 110ml of 5% NaOH solution was slowly added and neutralized. After extracting the Toluene layer, the solvent was removed to obtain a yellow solid. After recrystallization with Ethanol, 36g of compound 7 was obtained. (Yield 26%)
¹H NMR (500MHz, Acetone-D6, ppm): 7.2-7.35 (m, 40H), 5.3 (s, 16H), 4.7 (s, 16H), 4.2 (t, 4H), 3.7 (t, 4H), 3.4 (s, 4H)
HRMS [M+H]⁺: 1369.6258

### (8) Preparation of compound 8

Compound 8 was synthesized through the reaction shown in [Reaction Formula 8] below.

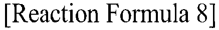

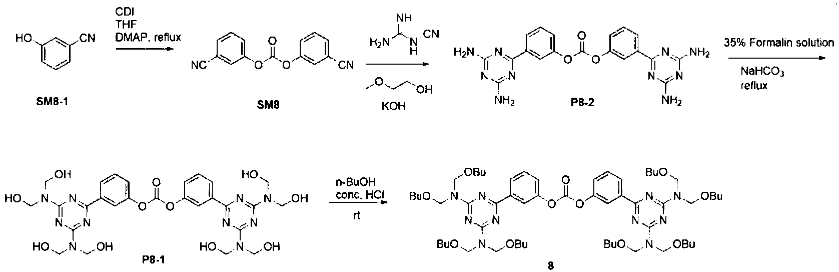

As shown in [Reaction Formula 8], in a 250ml round-bottom flask, 30g (0.25mol) of 3-cyanophenol (SM8-1, CAS: 873-62-1), 20g (0.12mol, 0.5eq) of carbonyl diimidazole (CDI, CAS:530-62-1), 1.5g (0.012mol, 0.05eq) of N,N-Dimethylaminopyridine (DMAP), and 120ml of Tetrahydrofuran (THF) were added and refluxed for 12 hours. After the reaction was completed, half of the solvent was removed, and 120ml of Ethanol was slowly added to form a solid, which was obtained through the filtration process to obtain 28.2g of SM8 compound.

Subsequently, as shown in [Reaction Formula 8], except for using 26g (0.1mol) of SM8 compound instead of (0.1mol) of Succinonitrile (SM1, CAS:110-61-2), P8-1 compound was synthesized by the same method as the preparation example of compound 1.

In a 250ml round-bottom flask, the obtained P8-1 compound and 200ml of n-butanol were added, and then 1.2ml (0.1 mol, 1eq) of 37% Hydrochloric acid was added. After raising the temperature to room temperature, it was stirred until completely dissolved. After the reaction was completed, 50ml of 5% NaOH solution was slowly added. After stirring for 1 hour, a solid was formed. After the filtration process, it was dried using an 80 °C vacuum oven, and 12.3g of compound 8 was obtained. (Yield 11%)
¹H NMR (500MHz, Acetone-D6, ppm): 8.1 (d, 2H), 7.4-7.5 (m, 6H), 5.2 (s, 16H) 3.4 (t, 16H), 1.4-1.5 (m, 32H), 1.05 (t, 24H)
HRMS [M+H]⁺: 1121.7288

### (9) Preparation of compound 9

Compound 9 was synthesized through the reaction shown in [Reaction Formula 9] below.

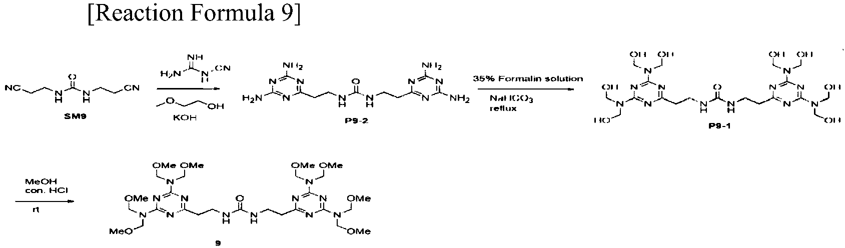

Specifically, as shown in [Reaction Formula 9], except for using 16.6g (0.1mol) of SM9 compound (CAS: 7253-99-8) instead of 8g (0.1mol) of Succinonitrile (SM1, CAS:110-61-2), 13.7g of compound 9 was obtained by the same method as the preparation example of compound 1. (Yield 20%)
¹H NMR (500MHz, Acetone-D6, ppm): 6.5 (brd, 2H), 5.3 (s, 16H), 3.7 (brd, 4H), 3.3 (s, 24H), 2.6 (t, 4H)
HRMS [M+H]⁺: 687.3851

### (10) Preparation of compound 10

Compound 10 was synthesized through the reaction shown in [Reaction Formula 10] below.

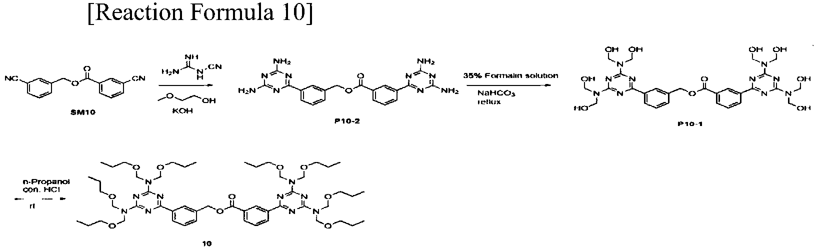

Specifically, as shown in [Reaction Formula 10], except for using 26.2g (0.1mol) of SM10 (CAS: 1090685-75-8) compound instead of 8g (0.1mol) of Succinonitrile (SM1, CAS:110-61-2), 15g of P10-1 compound was synthesized by the same method as the preparation example of compound 1.

In a 5000ml round-bottom flask, the obtained P10-1 compound and 200ml of n-propanol were added, and then 1.2ml (0.1 mol, 1eq) of 37% Hydrochloric acid was added. After raising the temperature to room temperature, it was stirred until completely dissolved. After the reaction was completed, 50ml of 5% NaOH solution was slowly added. After adding 50ml of water and stirring for 1 hour, a solid was formed. After the filtration process, it was dried using an 80 °C vacuum oven, and 18g of compound 10 was obtained. (Yield 18%)
¹H NMR (500MHz, Acetone-D6, ppm): 8.5-8.65 (m, 2H), 8.3 (d, 1H), 7.9-8.1 (m, 2H), 7.5-7.6 (m, 3H), 5.1-5.3 (m, 18H), 3.3-3.45 (m, 16H), 1.4-1.5 (m, 16H), 0.95-1.05 (t, 24H)
HRMS [M+H]⁺: 1007.6252

### (11) Preparation of compound 11

Compound 11 was synthesized through the reaction shown in [Reaction Formula 11] below.

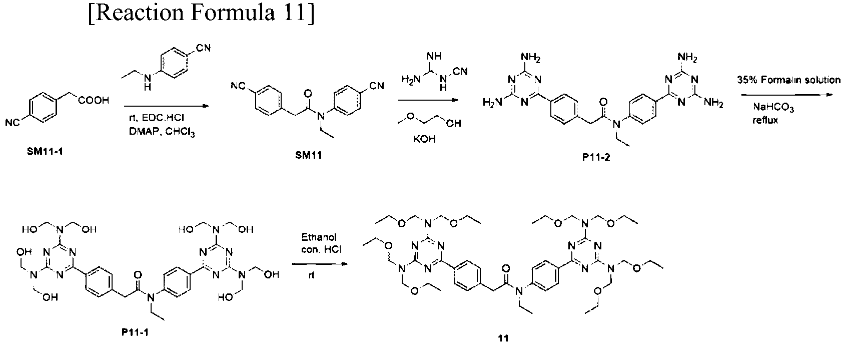

As shown in [Reaction Formula 11], in a 250ml round-bottom flask, 40g (0.25mol) of 4-Cyanophenylacetic acid (SM11-1, CAS: 5462-71-5), 36g (0.25mol, 1 eq) of 4-(Ethylamino)benzonitrile (CAS: 4714-63-0), 1.5g (0.012mol, 0.05eq) of N,N-Dimethylaminopyridine (DMAP), and 160ml of chloroform (CHCl₃) were added, and then 52.8g (0.28mol, 1.1eq) of EDC.HCl.2H₂O was slowly added. After stirring at room temperature for 12 hours, the reaction was terminated. Work-up was performed using saturated NH4Cl solution. After all CHCl₃ was removed, 120ml of Ethanol was added and stirred. The resulting solid was obtained through the filtration process to obtain 54.2g of SM11 compound.

Subsequently, as shown in [Reaction Formula 11], except for using 28.9g (0.1mol) of SM11 compound instead of (0.1mol) of Succinonitrile (SM1, CAS:110-61-2), P11-1 compound was synthesized by the same method as the preparation example of compound 1.

In a 250ml round-bottom flask, the obtained P11-1 compound and 100ml of Ethanol were added, and then 0.24ml (0.02mol, 0.2eq) of 37% Hydrochloric acid was added. After raising the temperature to room temperature, it was stirred until completely dissolved. After the reaction was completed, 50ml of 5% NaOH solution was slowly added and neutralized. After stirring for 1 hour, a solid was formed. After the filtration process, it was dried using an 80 °C vacuum oven, and 24g of compound 11 was obtained. (Yield 26%)
¹H NMR (500MHz, Acetone-D6, ppm): 8.6 (d, 2H), 8.17 (d, 2H), 7.66 (d, 2H), 7.4 (d, 2H), 5.2-5.4 (m, 16H), 4.4 (q, 2H), 4.05 (s, 2H), 3.4-3.5 (m, 16H), 1.3 (t, 3H), 0.95-1.1 (m, 24H)
HRMS [M+H]⁺: 922.5466

### (12) Preparation of compound 12

Compound 12 was synthesized through the reaction shown in [Reaction Formula 12] below.

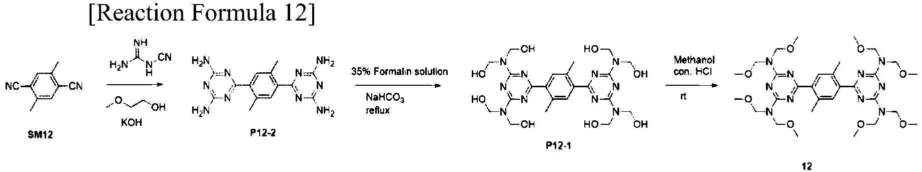

Specifically, as shown in [Reaction Formula 12], except for using 15.6g of SM12 (CAS: 39095-25-5) compound instead of 8g (0.1mol) of Succinonitrile (SM1, CAS:110-61-2), 14.8g of compound 12 was obtained by the same method as the preparation example of compound 1. (Yield 22%)
¹H NMR (500MHz, Acetone-D6, ppm): 7.9 (s, 2H), 5.2 (s, 16H), 3.3 (s, 24H), 2.5 (s, 6H)
HRMS [M+H]⁺: 677.3685

### <MANUFACTURING Example 2>: Synthesis of compound C1 to C2

### (1) Preparation of compound C1

Compound C1 was synthesized through the reaction shown in [Reaction Formula C1] below.

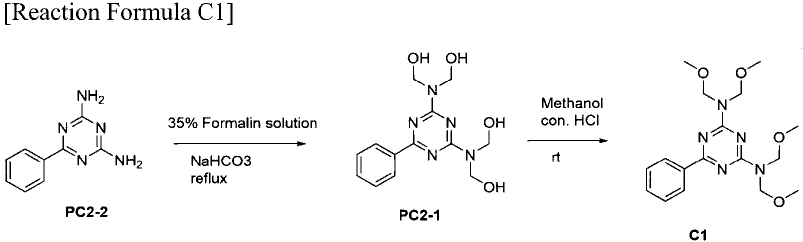

Specifically, as shown in [Reaction Formula C1], except for using 18.7g (0.1mol) of 2,4-Diamino-6-phenyl-1,3,5-triazine (PC2-2, CAS:91-76-9) compound instead of 8g (0.1mol) of Succinonitrile (SM1, CAS:110-61-2), 18g of compound C1 was obtained by the same method as the preparation example of compound 1. (Yield 22%)
¹H NMR (500MHz, Acetone-D6, ppm): 8.2 (d, 2H), 7.5-7.6 (m, 3H), 5.3 (s, 16H), 3.5 (s, 24H)
HRMS [M+H]⁺: 364.1937

### (2) Preparation of compound C2

Compound C2 was synthesized through the reaction shown in [Reaction Formula C2] below.

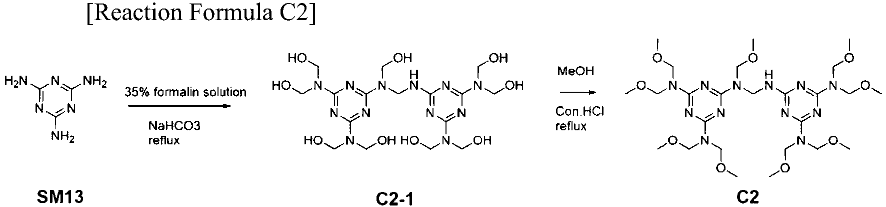

Specifically, as shown in [Reaction Formula C2], in a 250ml round-bottom flask, 12.3g (0.1 mol) of Melamine (SM13, CAS: 108-78-1), 0.84g (0.01mol, 0.1eq) of NaHCO₃, and 40ml (1mol, 5eq) of 35% formaldehyde aqueous solution were added and stirred at 80 °C for 12 hours. After the reaction was completed, the water was removed by vacuum distillation, and the remaining solid (C2-1) was used for the next reaction.

The obtained C2-1 compound, 80ml of Methanol was added and cooled to 5 °C, and then 0.24ml (0.02mol, 0.2eq) of 37% Hydrochloric acid was added. After raising the temperature to room temperature, it was stirred until completely dissolved. After the reaction was completed, 40ml of 5% NaOH solution was slowly added. After stirring for 1 hour, a solid was formed. After the filtration process, the obtained solid was recrystallized twice with Methanol/water (50ml/50ml) to obtain 5.5g of compound C2. (Yield 8.3%)
¹H NMR (500MHz, Acetone-D6, ppm): 6.05(s, 1H, N-H), 5.1-5.2(broad,18H), 4.9(s, 2H), 3.3-3.5(broad, 27H), 2.5(s, 4H)
HRMS [M+H]⁺: 661.3664

### <MANUFACTURING Example 3>: Synthesis of Polyimide Resin

In a round-bottom flask, 14.4g (0.31mol) of 2,2-bis(3-amino-4-hydroxyphenyl)hexafluoropropane (Bis-AP-AF) was dissolved in 500g of propylene glycol monomethyl ether acetate (PGMEA) at 60°C. Then, 91.1g (0.29mol) of 4,4'-oxydiphthalic anhydride (ODPA) was added and stirred for 2 hours, followed by the addition of 7.2g (0.03mol) of phthalic anhydride as an end-capping agent and stirred for an additional 2 hours. The temperature was then raised to 170°C and stirred for 6 hours. Afterward, it was cooled to room temperature. The weight average molecular weight (Mw) of the polymerized resin A measured by Gel Permeation Chromatography (GPC) was 15,000 g/mol, and the degree of imidization (DOI) of the resin A measured by infrared spectroscopy (IR) was 93%. The weight average molecular weight of the polymerized resin A was measured by Gel Permeation Chromatography in a tetrahydrofuran (THF) solvent.

### <MANUFACTURING Example 4>: Preparation of Photosensitive Resin Composition

The photosensitive resin composition was prepared by mixing the compositions described in Table 1 and Table 2 below and stirring for 24 hours. The values in Table 1 and Table 2 are the weight percentages of each component based on 100 weight% (solid content) of the polyimide resin.

**[Table 1]**

| Components | Type | Exampl e 1 | Exampl e 2 | Exampl e 3 | Exampl e 4 | Exampl e 5 | Exampl e 6 | Exampl e 7 |
|---|---|---|---|---|---|---|---|---|
| Resin | Polyimide of Manufacturin g Example 3 | 100 wt% | 100 wt% | 100 wt% | 100 wt% | 100 wt% | 100 wt% | 100 wt% |
| Photosensitiv e Compound (PAC) | MIPHOTO PAC425 | 15.50 wt% | 15.50 wt% | 15.50 wt% | 15.50 wt% | 15.50 wt% | 15.50 wt% | 15.50 wt% |
| Epoxy Curing Agent | PETG | 6 wt% | 6 wt% | 6 wt% | 6 wt% | 6 wt% | 6 wt% | 6 wt% |
| Crosslinking Agent | Compound 1 | 9 wt% | - | - | - | - | - | - |
| | Compound 2 | - | 9 wt% | - | - | - | - | - |
| | Compound 3 | - | - | 9 wt% | - | - | - | - |
| | Compound 4 | - | - | - | 9 wt% | - | - | - |
| | Compound 5 | - | - | - | - | 9 wt% | - | - |
| | Compound 6 | - | - | - | - | - | 9 wt% | - |
| | Compound 7 | - | - | - | - | - | - | 9 wt% |
| Solvent | PGMEA | 49.9 wt% | 49.9 wt% | 49.9 wt% | 49.9 wt% | 49.9 wt% | 49.9 wt% | 49.9 wt% |

**[Table 2]**

| Component s | Type | Examp le 8 | Examp le 9 | Examp le 10 | Examp le 11 | Examp le 12 | Comparati ve Example 1 | Comparati ve Example 2 |
|---|---|---|---|---|---|---|---|---|
| Resin | Polyimide of Manufacturi ng Example 3 | 100 wt% | 100 wt% | 100 wt% | 100 wt% | 100 wt% | 100 wt% | 100 wt% |
| Photosensiti ve Compound (PAC) | MIPHOTO PAC425 | 15.50 wt% | 15.50 wt% | 15.50 wt% | 15.50 wt% | 15.50 wt% | 15.50 wt% | 15.50 wt% |
| Epoxy Curing Agent | PETG | 6 wt% | 6 wt% | 6 wt% | 6 wt% | 6 wt% | 6 wt% | 6 wt% |
| Crosslinkin g Agent | Compound 8 | 9 wt% | - | - | - | - | - | - |
| | Compound 9 | - | 9 wt% | - | - | - | - | - |
| | Compound 10 | - | - | 9 wt% | - | - | - | - |
| | Compound 11 | - | - | - | 9 wt% | - | - | - |
| | Compound 12 | - | - | - | - | 9 wt% | - | - |
| | Compound C1 | - | - | - | - | - | 9 wt% | - |
| | Compound C2 | - | - | - | - | - | - | 9 wt% |
| Solvent | PGMEA | 49.9 wt% | 49.9 wt% | 49.9 wt% | 49.9 wt% | 49.9 wt% | 49.9 wt% | 49.9 wt% |

In Table 1 and Table 2 above,
MIPHOTO PAC425 refers to NAC5 ester with 2,3,4,4'-tetrahydroxy benzophenone from Miwon commercial co., Ltd, and
PETG refers to Pentaerythritol tetraglycidyl ether.

### <EXPERIMENTAL Example>

The properties of the compound or cured layer obtained in the examples and comparative examples were measured by the following methods, and the results are shown in Table 3.

### 1. Formaldehyde Index

The Formaldehyde index of the compound obtained in the examples and comparative examples was measured according to EPA (US Environmental Protection Agency) Method 8315A (SW-846).

### 2. Young's Modulus, Tensile Strength, and Elongation

The photosensitive resin composition of Manufacturing Example 4 was spin-coated on a 6-inch wafer to form a film, the solvent was evaporated at 120°C for 2 minutes, and it was initially cured. It was then finally cured at 180 °C for 2 hours in a nitrogen atmosphere oven (Koyo INH oven), and the thickness of the final cured layer was 10 um. To remove the cured layer from the wafer, it was soaked in 2.5% HF diluted with DI-water (deionized water) for 30 minutes, and when the film floated, it was taken out and washed three times with deionized water (DI-water). The obtained cured layer was dried in a convection oven at 40 °C for 1 hour and then cut into a size of 10cm × 1cm to prepare a sample for measuring properties.

At 25 °C, the prepared sample was loaded into a UTM (Universal Testing Machine, Zwick) equipment, and Young's modulus, tensile strength, and elongation were measured. The measurement length of the sample was set to 1cm x 5cm, the pulling speed was set to 10.0 mm/min, and the load cell was 0.5 kN.

### 3. Glass Transition Temperature (Tg) and Coefficient of Thermal Expansion (CTE)

The photosensitive resin composition of Manufacturing Example 4 was spin-coated on a 6-inch wafer to form a film, the solvent was evaporated at 120°C for 2 minutes, and it was initially cured. It was then finally cured at 180 °C for 2 hours in a nitrogen atmosphere oven (Koyo INH oven), and the thickness of the final cured layer was 10 um. To remove the cured layer from the wafer, it was soaked in 2.5% HF diluted with DI-water (deionized water) for 30 minutes, and when the film floated, it was taken out and washed three times with deionized water (DI-water). The obtained cured layer was dried in a convection oven at 40 °C for 1 hour and then cut into a size of 10cm × 1cm to prepare a sample for measuring properties.

The prepared sample was heated at a rate of 10°C/min under a nitrogen atmosphere from a temperature of 25 °C using a TA Q400 equipment to measure the glass transition temperature and coefficient of thermal expansion.

**[Table 3]**

| Experimental Results | | | | | | |
|---|---|---|---|---|---|---|
| | Formaldehyde Index (ppm) | Young's Modulus (MPa) | Tensile Strength (MPa) | Elongation (%) | CTE (ppm/°C ) | Tg (°C) |
| Example 1 | 25 | 3489 | 126 | 31 | 41 | 292 |
| Example 2 | 26 | 3501 | 130 | 29 | 35 | 305 |
| Example 3 | 20 | 3520 | 115 | 16 | 19 | 350 |
| Example 4 | 18 | 3515 | 117 | 17 | 20 | 355 |
| Example 5 | 21 | 3480 | 124 | 26 | 39 | 295 |
| Example 6 | 23 | 3499 | 127 | 27 | 30 | 310 |
| Example 7 | 32 | 3508 | 131 | 35 | 41 | 268 |
| Example 8 | 29 | 3512 | 121 | 19 | 21 | 340 |
| Example 9 | 37 | 3495 | 124 | 32 | 42 | 299 |
| Example 10 | 24 | 3500 | 119 | 22 | 29 | 320 |
| Example 11 | 19 | 3505 | 118 | 21 | 33 | 315 |
| Example 12 | 17 | 3517 | 125 | 20 | 31 | 325 |
| Comparative Example 1 | 40 | 3510 | 120 | 10 | 43 | 265 |
| Comparative Example 2 | 110 | 3501 | 119 | 15 | 60 | 270 |

As shown in Table 1, it was confirmed that the compound of the examples had a lower Formaldehyde index compared to the comparative examples. Additionally, it was confirmed that the cured layer applied with the compound of the examples had superior mechanical properties and thermal properties compared to the comparative examples.

## Claims

1. A compound represented by the following chemical formula 1: 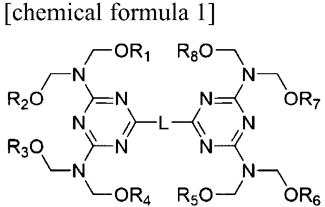 In the chemical formula 1 above,
R₁ to R₈ are the same or different from each other, each independently being one of alkyl, arylalkyl, or alkoxyarylalkyl, and
L is a functional group represented by the following chemical formula 2, 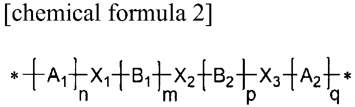 in the chemical formula 2 above,
X₁, X₂, X₃ are the same or different from each other, each independently being one of direct bond, -O-, -COO-, -CONR₉-, -O(CO)O-, or -NR₁₀(CO)NR₁₁-, and
R₉ to R₁₁ are the same or different from each other, each independently being one of hydrogen, alkyl, cycloalkyl, or aryl, and
A₁, A₂, B₁, and B₂ are the same or different from each other, each independently being one of alkylene, cycloalkylene, or arylene, and
n, m, p, and q are the same or different from each other, each independently being an integer of 0 or 1, and at least one of n, m, p, and q is 1.

2. The compound according to claim 1, wherein A₁, A₂, B₁, and B₂ are the same or different from each other, each independently being one of methylene, ethylene, normal propylene, 1,3-butandiyl, cyclohexylene, 1,3-phenylene, 5-methyl-1,3-phenylene, 2,5-dimethyl-1,4-phenylene, 1,5-naphthylene, or 1,6-naphthylene.

3. The compound according to claim 1, wherein the functional group represented by the chemical formula 2 is any one selected from the group consisting of the following:

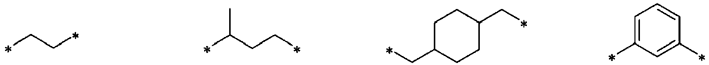

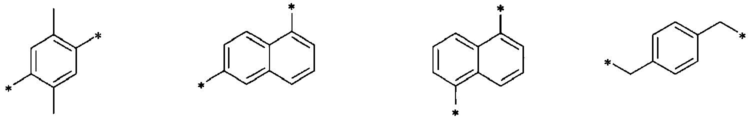

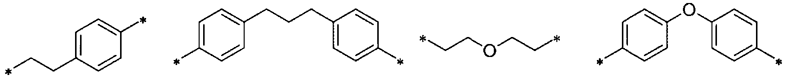

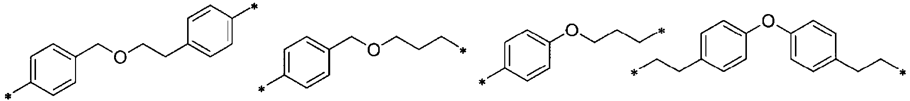

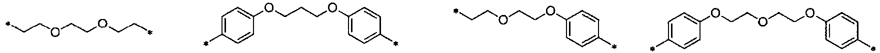

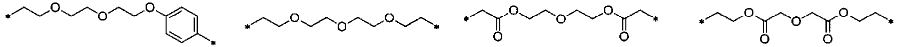

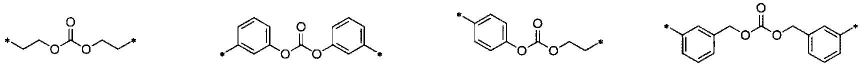

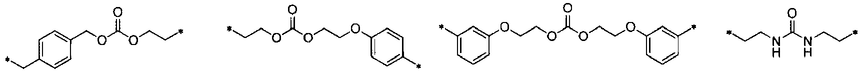

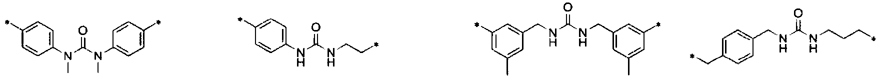

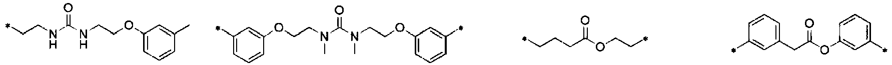

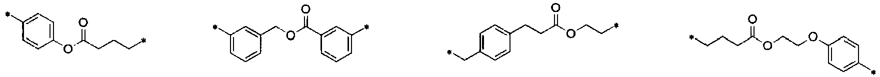

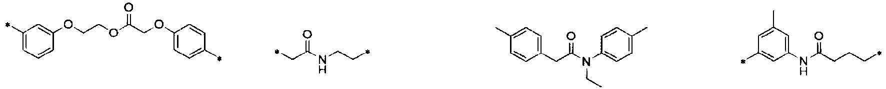

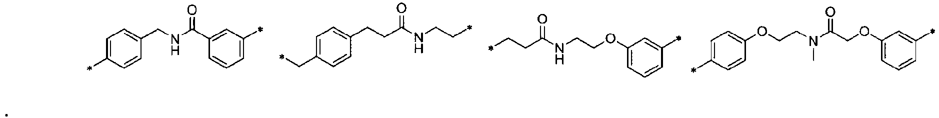

4. The compound according to claim 1, wherein at least one of X₁, X₂, X₃ in the chemical formula 2 is one of -O-, -COO-, -CONR₉-, -O(CO)O-, or -NR₁₀(CO)NR₁₁-.

5. The compound according to claim 1, wherein R₉ is one of hydrogen, methyl, or ethyl.

6. The compound according to claim 1, wherein R₁₀ and R₁₁ are the same or different from each other, each independently being one of hydrogen or methyl.

7. The compound according to claim 1, wherein (n+m+p+q) in the chemical formula 2 is an integer from 2 to 4.

8. The compound according to claim 1, wherein in the chemical formula 1, R₁ to R₈ are the same or different from each other, each independently being one of alkyl with 1 to 5 carbon atoms, arylalkyl with 7 to 20 carbon atoms, or alkoxyarylalkyl with 8 to 30 carbon atoms.

9. The compound according to claim 1, wherein in the chemical formula 1, R₁ to R₈ are the same or different from each other, each independently being one of methyl, ethyl, normal propyl, isopropyl, normal butyl, benzyl, or 4-methoxybenzyl.

10. The compound according to claim 1, wherein in the chemical formula 1, R₁ to R₈ are the same.

11. The compound according to claim 1, wherein the compound represented by the chemical formula 1 comprises a compound represented by any one of chemical formula 1-1 to chemical formula 1-12:

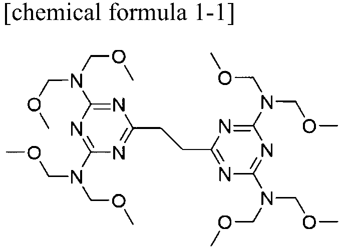

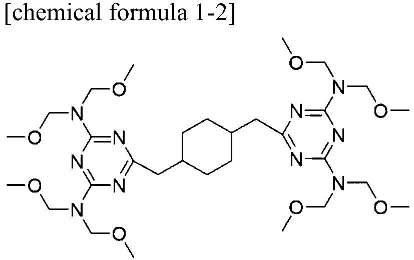

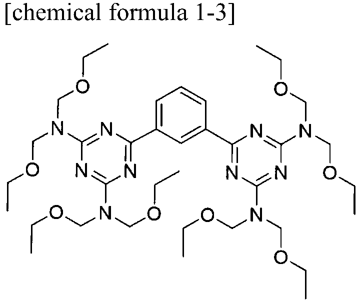

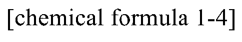

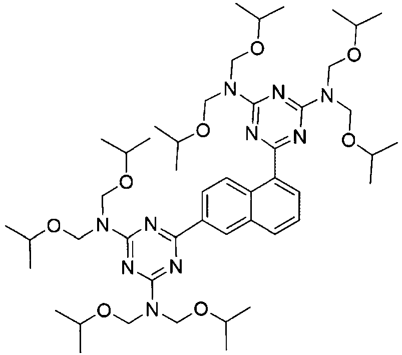

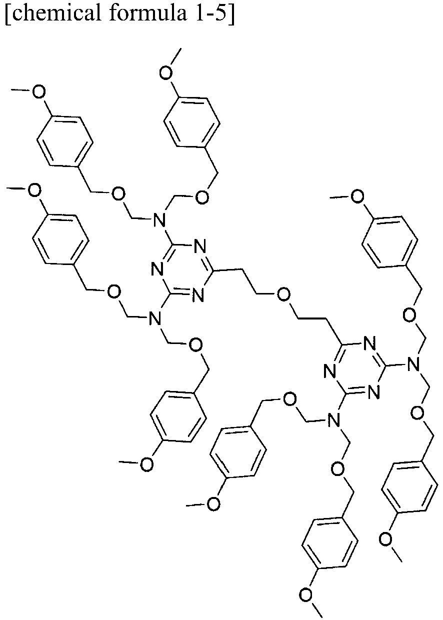

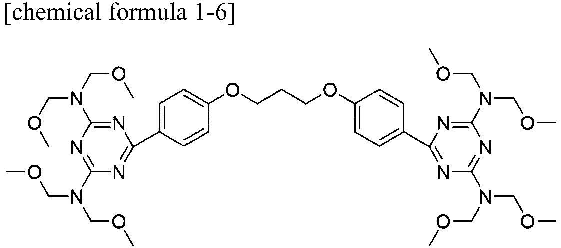

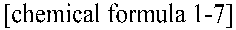

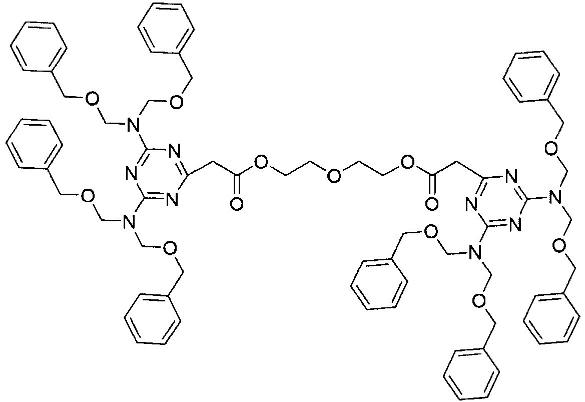

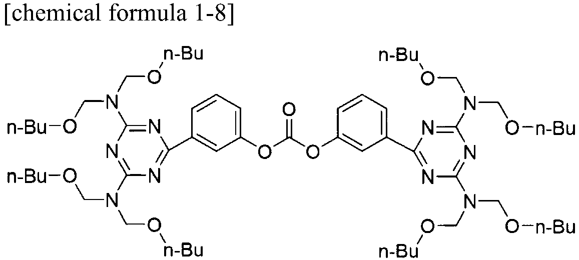

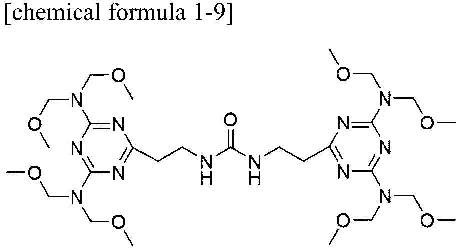

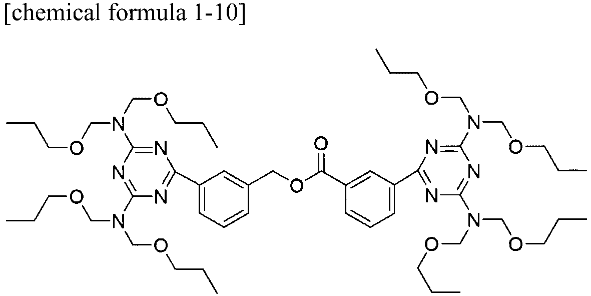

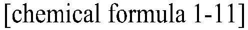

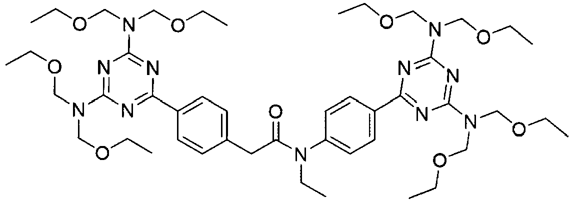

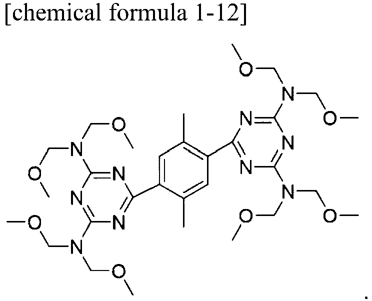

12. The compound according to claim 1, wherein the Formaldehyde index according to EPA Method 8315A is 38 ppm or less.

13. A method for preparing a compound comprising the steps of reacting a compound represented by chemical formula 3 with dicyandiamide to produce a compound represented by chemical formula 4; reacting the compound represented by chemical formula 4 with formaldehyde to produce a compound represented by chemical formula 5; and reacting the compound represented by chemical formula 5 with alcohol:
[chemical formula 3] NC-L-CN 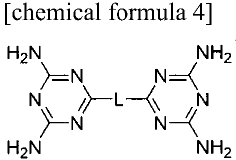 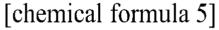 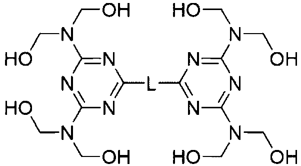
in the chemical formula 3 to 5 above,
L is a functional group represented by the following chemical formula 2, 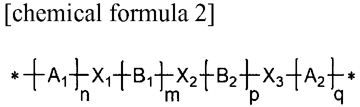 in the chemical formula 2 above,
X₁, X₂, X₃ are the same or different from each other, each independently being one of direct bond, -O-, -COO-, -CONR₉-, -O(CO)O-, or -NR₁₀(CO)NR₁₁-, and
R₉ to R₁₁ are the same or different from each other, each independently being one of hydrogen, alkyl, cycloalkyl, or aryl, and
A₁, A₂, B₁, and B₂ are the same or different from each other, each independently being one of alkylene, cycloalkylene, or arylene, and
n, m, p, and q are the same or different from each other, each independently being an integer of 0 or 1, and at least one of n, m, p, and q is 1.

14. The method according to claim 13, wherein the alcohol comprises a compound represented by the following chemical formula 6:
[chemical formula 6] R₁₃-OH
in the chemical formula 6 above,
R₁₃ is one of alkyl, arylalkyl, or alkoxyarylalkyl.

15. A crosslinking agent comprising the compound according to claim 1.

16. A photosensitive resin composition comprising the compound according to claim 1.

17. A cured layer comprising the cured product of the photosensitive resin composition according to claim 16.

18. An electronic device comprising the cured layer according to claim 17.
